# EUROPEAN PATENT APPLICATION

(11) **EP 4 144 860 A1**
(43) Date of publication of application: **08.03.2023**
(21) Application number: 21795393.4
(22) Date of filing: 29.04.2021
(51) Int. Cl.: C12Q 1/68, G01N 33/543, G01N 33/53

(54) **CHIP SURFACE LINKER AND PREPARATION METHOD AND USE THEREFOR**

(30) Priority: 30.04.2020 CN 202010366021
(71) Applicant: Nanjing GenScript Biotech Co., Ltd., Nanjing, Jiangsu 211100 (CN)
(72) Inventor: HU, Feichi, Nanjing, Jiangsu 211100 (CN); WU, Cheng-Hsien, Nanjing, Jiangsu 211100 (CN); WANG, Jianpeng, Nanjing, Jiangsu 211100 (CN); FAN, Yufeng, Nanjing, Jiangsu 211100 (CN); WANG, Mengjia, Nanjing, Jiangsu 211100 (CN)
(74) Representative: Wichmann, Hendrik
(86) International application number: PCT/CN2021/090905
(87) International publication number: WO 2021/219073

(57) **Abstract**

The present invention relates to the field of biochips, and provides a chip surface linker and a preparation method and a use therefor. The chip surface linker is obtained by means of applying a direct current voltage to an aromatic amine bonding molecule in the presence of an acid and a nitrite to cause a reaction with a chip surface to form a bonding molecular group connected the chip surface, and then using a functional molecular for reaction and modification to add a functional molecular group containing a hydroxyl group and an ester group. The chip surface linker obtained in the present invention is able to bond more stably with a chip surface, being stable in hot water and basic conditions, and features relatively good electrical conductivity, stability during energization, and resistance to organic solvents required for nucleic acid synthesis, and is thus very advantageous for subsequent nucleic acid synthesis and other uses.

## Description

### Technical Field

The present invention relates to the technical field of biochip preparation, in particular to a linker used for an electrically assisted chip synthesis of nucleic acid, and a preparation method and a use therefor.

### Background Art

Stable linkers are very important for metal or semiconductor chips. On one hand, stable linkers can be used for in-situ synthesis or pre-preparation of a large number of oligonucleotide polymers (oligo pool) and DNA probes on the surface so as to ensure that nucleic acid molecules do not fall down in the synthesis process, thereby ensuring the synthesis quality. On the other hand, stable linkers can firmly connect the nucleic acid on the chip surface to achieve different uses, such as in-situ hybridization on the chip, chip screening and chip diagnosis; and by detecting and analyzing a sample hybridization signal, qualitative or quantitative analysis can be carried out on a specific biomarker in a sample, which can play a huge role in disease diagnosis, drug screening, new drug development, agricultural and environmental research and other fields. However, there are still some problems with existing linkers used for synthesizing nucleic acids on chips, especially in terms of linker stability and preparation costs. Those problems are associated with methods for preparing the linkers. The methods for preparing the linkers in the prior art mainly include small molecule adhesion [1], metal-sulfydryl reaction [2] and polymer coating [3,4]. Among them, the small molecule adhesion uses the adhesion of high-concentration small molecules to form a linker on the chip surface. The disadvantage of this method is that the prepared linker has weak adhesion, especially may fall off in the process of nucleic acid synthesis, resulting in increased synthesis errors. In addition, the linker prepared by this method is sensitive to water and will fall off during subsequent applications, which makes it difficult to further meet the application needs of our customers. The metal-sulfhydryl reaction uses a sulfhydryl to react with a metal to form a covalent bond as a linker. The problem of this method is that when synthesizing nucleic acids by means of electrification, the sulfhydryl will be reduced and fall off from the metal surface, resulting in the failure of nucleic acid synthesis and making it more difficult for subsequent applications. The polymer coating uses a polymer or nanomaterial to coat the chip surface and then modifies the corresponding molecule to form a linker. However, the combination stability of the coating material and the metal surface is limited, the thickness of coating is difficult to control each time, the highly uniform surface is difficult to achieve and the stability in hot water or basic conditions is still insufficient. There is an urgent need to solve these problems.

### Summary of the Invention

In view of this, the present application provides a chip surface linker and a preparation method and a use therefor. The chip surface linker provided by the present application has good stability, is stable in hot water and basic conditions, has good electrical conductivity, stability during energization and resistance to organic solvents required for nucleic acid synthesis, and is advantageous for nucleic acid synthesis and the like.

The present invention provides a chip surface linker, and the linker is obtained by the following steps: step 1: applying a direct current voltage to cause an aromatic amine bonding molecule to react with a chip surface in the presence of an acid and a nitrite to form a bonding molecule group connected to the chip surface; and step 2: using a functional molecule for reaction and modification to obtain a linker containing a functional molecule group, wherein the functional molecule group contains a hydroxyl group and an ester group.

In some embodiments, the bonding molecule is an aniline substance. In some other embodiments, the bonding molecule is selected from p-aminophenylacetic acid, p-aminophenylethanol or p-aminophenylenediamine, preferably p-aminophenylacetic acid.

In some embodiments, the direct current voltage is a constant direct current voltage, and the direct current voltage applied is selected from 0.5 to 5.0 V, preferably 2.5 to 3.0 V, more preferably 2.5 V. Other preferred constant direct current voltages are 0.5 V, 0.6 V, 0.7 V, 0.8 V, 0.9 V, 1.0 V, 1.1 V, 1.2 V, 1.3 V, 1.4 V, 1.5 V, 1.6 V, 1.7 V, 1.8 V, 1.9 V, 2.0 V, 2.1 V, 2.2 V, 2.3 V, 2.4 V, 2.6 V, 2.7 V, 2.8 V, 2.9 V, 3.0 V, 3.1 V, 3.2 V, 3.3 V, 3.4 V, 3.5 V, 3.6 V, 3.7 V, 3.8 V, 3.9 V, 4.0 V, 4.1 V, 4.2 V, 4.3 V, 4.4 V, 4.5 V, 4.6 V, 4.7 V, 4.8 V, 4.9 V or 5.0 V.

In some embodiments, the direct current voltage is applied for 10 to 50 min, preferably 10 to 30 min, more preferably 20 min. Other preferred direct current voltage application time is 10 min, 12 min, 14 min, 16 min, 18 min, 20 min, 22 min, 24 min, 26 min, 28 min or 30 min.

In some embodiments, the nitrite is selected from sodium nitrite, potassium nitrite or calcium nitrite, preferably sodium nitrite.

In some embodiments, the acid is selected from hydrochloric acid, nitric acid or sulfuric acid, preferably hydrochloric acid.

In some embodiments, the functional molecule is a hydroxyl substance containing a long carbon chain and an ester group. In some other embodiments, the functional molecule is selected from a succinic anhydride modified base monomer, hydroxyethyl methacrylate, a succinic acid modified base monomer or an oxalic acid modified base monomer, preferably a succinic anhydride modified base monomer. In some embodiments, the base monomer in the functional molecule is selected from one or more of adenine, guanine, cytosine, thymine and uracil. In a specific embodiment, the functional molecule is succinic anhydride modified adenine, succinic anhydride modified guanine, succinic anhydride modified cytosine, succinic anhydride modified thymine or succinic anhydride modified uracil.

In some embodiments, a connecting arm molecule group is further included between the bonding molecule group and the functional molecule group. Preferably, the connecting arm molecule group is reacted with and connected to the bonding molecule group by a connecting arm molecule, and the functional molecule group is reacted with and connected to the connecting arm molecule group by a functional molecule.

In some embodiments, the connecting arm molecule is a diamine or diol substance. In some other embodiments, the connecting arm molecule is selected from ethylenediamine, hexamethylenediamine, decanediamine, 1,8-octanediamine, ethylene glycol, hexanediol, decanediol or 1,8-octanediol, preferably 1,8-octanediamine. In some embodiments, the connecting arm molecule is a diamine substance. In some other embodiments, the connecting arm molecule is selected from ethylenediamine, hexanediamine, decanediamine or 1,8-octanediamine, preferably 1,8-octanediamine.

In some embodiments, the chip is a metal chip, preferably a gold chip, a platinum chip or an aluminum chip, more preferably a metal platinum chip.

The present invention further provides a preparation method of a chip surface linker, comprising the following steps:
step 1: mixing an aromatic amine bonding molecule with an acid and a nitrite to obtain a mixed solution;
step 2: bringing the mixed solution in step 1 into contact with a chip surface, and applying a direct current voltage for reaction to form a bonding molecule group connected to the chip surface; and
step 3: using a functional molecule for reaction and modification to obtain a linker containing a functional molecule group, wherein the functional molecule group contains a hydroxyl group and an ester group.

In some embodiments, before step 3, the preparation method comprises bringing the reacted chip surface in step 2 into contact with a connecting arm molecule for reaction, so as to connect a connecting arm molecule group. The reacted chip surface is further contacted with the functional molecule in step 3 for reaction so as to connect the functional molecule group.

In some embodiments, the bonding molecule is an aniline substance. In some other embodiments, the bonding molecule is p-aminophenylacetic acid, p-aminophenylethanol or p-aminophenylenediamine, preferably p-aminophenylacetic acid.

In some embodiments, the functional molecule is a hydroxyl substance containing a long carbon chain and an ester group. In some other embodiments, the functional molecule is selected from a succinic anhydride modified base monomer, hydroxyethyl methacrylate, a succinic acid modified base monomer or an oxalic acid modified base monomer, more preferably a succinic anhydride modified monomer, wherein the base monomer in the functional molecule is selected from one or more of adenine, guanine, cytosine, thymine and uracil.

In some embodiments, the direct current voltage in step 2 is a constant direct current voltage, and the direct current voltage applied is selected from 0.5 to 5.0 V, preferably 0.5 to 3.0 V, more preferably 2.5 V.

In some embodiments, in step 2, when the mixed solution obtained in step 1 is brought into contact with the chip surface and the constant direct current voltage is applied for reaction, the temperature is 4°C to 40°C, preferably 20°C to 37°C, such as 20°C, 21°C, 22°C, 23°C, 24°C, 25°C, 26°C, 27°C, 28°C, 29°C, 30°C, 31°C, 32°C, 33°C, 34°C, 35°C, 36°C or 37°C, preferably room temperature; and the reaction time is 10 to 50 min, preferably 10 to 30 min, such as 10 min, 12 min, 14 min, 16 min, 18 min, 20 min, 22 min, 24 min, 26 min, 28 min or 30 min, more preferably 20 min.

In some embodiments, the nitrite is selected from sodium nitrite, potassium nitrite or calcium nitrite, preferably sodium nitrite.

In some embodiments, the acid is selected from hydrochloric acid, nitric acid or sulfuric acid, preferably hydrochloric acid.

In some embodiments, the connecting arm molecule is a diamine or diol substance selected from ethylenediamine, hexamethylenediamine, decanediamine, 1,8-octanediamine, ethylene glycol, hexanediol, decanediol or 1,8-octanediol, preferably 1,8-octanediamine. In some embodiments, the connecting arm molecule is a diamine substance selected from ethylenediamine, hexanediamine, decanediamine or 1,8-octanediamine, preferably 1,8-octanediamine.

In some embodiments, the chip is a metal chip selected from a gold chip, a platinum chip and an aluminum chip, preferably a metal platinum chip.

The present invention further provides the use of the chip surface linker described above in nucleic acid synthesis or chip kit preparation.

### Detailed Description of the Invention

The present invention provides a chip surface linker. A direct current voltage is applied to cause an aromatic amine bonding molecule to react with a chip surface in the presence of an acid and a nitrite to form a bonding molecule group connected to the chip surface, then a connecting arm molecule and the bonding molecule group are reacted and connected to form a connecting arm molecule group, and next, a functional molecule is reacted with the connecting arm molecule group to obtain a linker containing a functional molecule group, wherein the functional molecule group contains a hydroxyl group and an ester group.

The bonding molecule described in the present invention is an aromatic amine substance, such as an aniline substance, which may be p-aminophenylacetic acid, p-aminophenethyl alcohol or p-aminophenylenediamine. In some embodiments of the present invention, the bonding molecule is p-aminophenylacetic acid. The bonding molecule group described in the present invention is a group that is covalently connected to the chip surface after the bonding molecule and the chip surface undergo an electrically promoted reaction. In some embodiments, the bonding molecule group is a group formed by applying a constant direct current voltage to cause the aromatic amine bonding molecule to react with the chip surface in the presence of an acid and a nitrite. In some embodiments, the bonding molecule may be p-aminophenylacetic acid, p-aminophenylethanol or p-aminophenylenediamine. In the present invention, the aromatic amine bonding molecule is diazotized to generate aromatic carbon radicals in the presence of an acid and a nitrite by applying constant direct current voltage, and the aromatic carbon radicals and chip electrode materials are reacted to form covalent bonds and bonded.

The connecting arm molecule described in the present invention is a diamine or diol substance, which may be ethylenediamine, hexamethylenediamine, decanediamine, 1,8-octanediamine, ethylene glycol, hexanediol, decanediol or 1,8-octanediol. In some embodiments of the present invention, the connecting arm molecule is 1,8-octanediamine. The connecting arm molecule group described in the present invention is a group generated by reacting the connecting arm molecule with the bonding molecule group and the functional molecule sequentially. In some embodiments, the connecting arm molecule group may be selected from ethylenediamine group, hexamethylenediamine group, decanediamine group, 1,8-octanediamine group, ethylene glycol group, hexanediol group, decanediol group or 1,8-octanediol group. In the present invention, the chip connected with the bonding molecule group is immersed in a connecting arm molecule solution, thereby modifying a long-chain molecule, increasing the distance between the functional group and the chip, and reducing the steric hindrance in subsequent use.

In the present invention, the functional molecule is a hydroxy substance containing a long carbon chain and an ester group, and may be a succinic anhydride modified base monomer, hydroxyethyl methacrylate, a succinic acid modified base monomer or an oxalic acid modified base monomer. In some embodiments of the present invention, the functional molecule is a mixed solution of two or more of succinic anhydride modified adenine, succinic anhydride modified guanine, succinic anhydride modified cytosine or succinic anhydride modified thymine. In some embodiments of the present invention, succinic anhydride modified adenine, succinic anhydride modified guanine, succinic anhydride modified cytosine or succinic anhydride modified thymine solution is used alone. In some embodiments, the functional molecule group may be selected from groups with hydroxyl group removed after reacting carboxylic acid and an amino group in succinic anhydride modified adenine, succinic anhydride modified guanine, succinic anhydride modified cytosine, succinic anhydride modified thymine or succinic anhydride modified uracil, hydroxyethyl methacrylate, succinic acid modified adenine, succinic acid modified guanine, succinic acid modified cytosine, succinic acid modified thymine or succinic acid modified uracil, and oxalic acid modified adenine, oxalic acid modified guanine, oxalic acid modified cytosine, oxalic acid modified thymine or oxalic acid modified uracil. In the present invention, the functional molecule is modified on the connecting arm, and the formed functional molecule group contains a hydroxyl group and an ester group, which facilitates the subsequent electrically promoted DNA synthesis and cleavage on the chip.

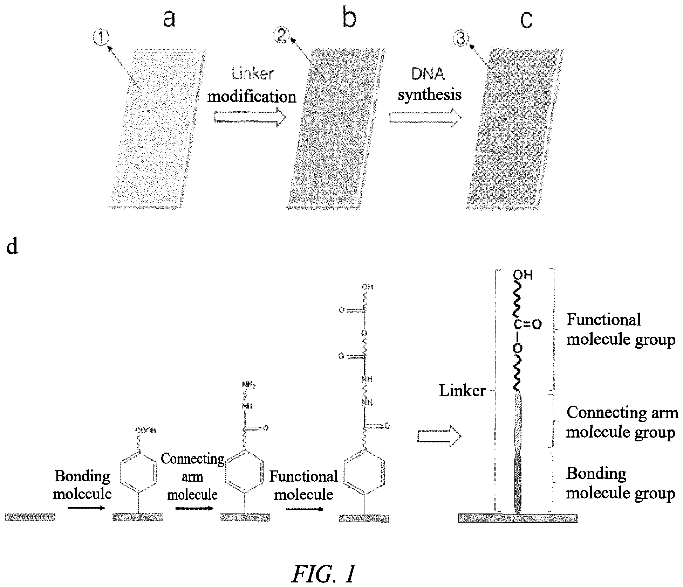

The chip surface linker of the present invention comprises: (1) a bonding molecule group, which is covalently connected to the chip surface; (2) a functional molecule group, which includes reactive groups such as a hydroxyl group and an ester group; and (3) a connecting arm molecule group connecting the bonding molecule group to the functional molecule group. FIGs. 1a-1d are schematic diagrams of preparation of a chip linker and DNA synthesis according to an embodiment of the present invention, wherein in FIG.1d, the chip surface is sequentially connected to a bonding molecule, a connecting arm molecule and a functional molecule to obtain a chip surface linker, and the chip surface linker comprises a bonding molecule group, a connecting arm molecule group and a functional molecule group. The bonding molecule sequentially connected to the chip surface linker described in the present invention is p-aminophenylacetic acid, the connecting arm molecule is 1,8-octanediamine, and the functional molecule is succinic anhydride modified adenine, succinic anhydride modified guanine, succinic anhydride modified cytosine or succinic anhydride modified thymine.

The chip surface linker described in the present invention is stably bonded to the chip surface through covalent bonds, and can be used for applications such as electric-assisted chip synthesis of nucleic acids. The metal is generally a sheet metal material, that is, a metal chip, and the metal component may be selected from gold, platinum or aluminum. In some embodiments of the present invention, the metal chip is a platinum chip. In a specific embodiment of the present invention, the chip is a CustomArray chip. In the embodiments of the present invention, the metal sheet can be rinsed with water and alcohol in turn, then soaked in an acid solution, heated to a certain temperature such as 40-70°C, placed for 5 to 30 min, and finally rinsed with water and dried to obtain a dry and clean metal surface, wherein the water is generally distilled water, the alcohol is ethanol, and the acid solution mainly uses prinaha solution (volume ratio of H₂SO₄ : H₂O₂ = 3:1), which mainly removes pollutants such as other impurities of metal dust, inorganic particles and organic small molecules.

The present invention further provides a preparation method of a chip surface linker, comprising the following steps: mixing a bonding molecule with hydrochloric acid and a sodium nitrite solution to obtain a mixed solution; then, bringing a chip surface in contact with the mixed solution, and applying a constant direct current voltage for reaction; and bringing the reacted chip surface in contact with a connecting arm molecule solution and a functional molecule solution in turn for reaction and modification to prepare the linker, as shown in FIG. 1d. In an embodiment of the present invention, a clean metal chip is first provided, a mixed solution is obtained by mixing a bonding molecule with hydrochloric acid and a sodium nitrite solution, and finally the metal chip is brought into contact with the mixed solution for reaction.

In some embodiments of the present invention, preferably 15 mM hydrochloric acid is used as a solvent, a cold bonding molecule is mixed with the solvent, sodium nitrite is then added, and same is quickly shaken well to prepare a mixed solution. The bonding molecule is an aromatic amine substance, preferably an aniline substance, including but not limited to one or more of p-aminophenylacetic acid, p-aminophenylethanol and p-aminophenylenediamine. In some embodiments of the present invention, the bonding molecule is p-aminophenylacetic acid. In one embodiment of the present invention, 0.10-0.15 mM cold p-aminophenylacetic acid and 15 mM hydrochloric acid are mixed uniformly, 0.07-0.10 mM sodium nitrite is then added, and same is quickly shaken well to prepare a mixed solution. The cleaned metal chip is quickly immersed in the above mixed solution, that is, the metal surface is brought into contact with the mixed solution, a constant direct current voltage is applied and same is left to stand for reaction. In some embodiments of the present invention, the constant direct current voltage applied is 0.5 V to 3.0 V, preferably 2.5 V; the reaction temperature is 20°C to 37°C, preferably room temperature; and the reaction time is 10 to 30 min, preferably 20 min. In some embodiments of the present invention, the constant direct current voltage applied to the chip is 0.5 V to 3.0 V, and the chip is left to stand at room temperature for electrically promoted reaction for 10 to 30 minutes. In a specific embodiment of the present invention, the constant direct current voltage applied to the chip is 2.5 V, and the chip is left to stand for an electrically promoted reaction at room temperature for 20 min. The principle of this step is that the aromatic amine molecule is diazotized to generate aromatic carbon radicals in the presence of hydrochloric acid and sodium nitrite by applying constant direct current voltage, and the aromatic carbon radicals and electrode materials are reacted to form covalent bonds and bonded. The specific reaction is as follows: 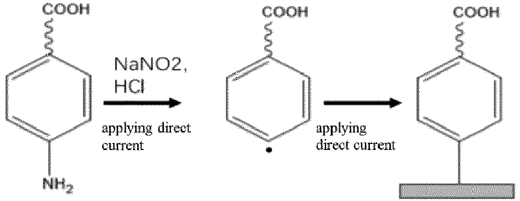 after reaction, the chip is taken out, washed with water and alcohol in turn, then rinsed with an acid solution, and finally rinsed with water and blow-dried.

The cross-linking acylation reaction of the carboxyl and the amino group that occurs on the reacted chip can modify the connecting arm molecule, thereby increasing the distance between the functional group and the chip and reducing the steric hindrance in subsequent use. In some embodiments of the present invention, the connecting arm molecule is a diamine substance, including but not limited to one or more of ethylenediamine, hexamethylenediamine, decanediamine and 1,8-octanediaminemethanol. In some embodiments of the present invention, the connecting arm molecule is 1,8-octanediamine. The reacted chip is soaked in a 1,8-octanediaminemethanol solution, left to stand for reaction for 8 to 12 h, preferably 8 h, and then washed with water and blow-dried.

The reacted chip is then modified with a functional molecule. The functional molecule is a hydroxyl substance containing a long carbon chain and an ester group, and includes but not limited to a succinic anhydride modified base monomer, hydroxyethyl methacrylate, a succinic acid modified base monomer or an oxalic acid modified base monomer. In some embodiments of the present invention, a solution of succinic anhydride modified adenine, succinic anhydride modified guanine, succinic anhydride modified cytosine, succinic anhydride modified thymine or succinic anhydride modified uracil is used alone. In other embodiments of the present invention, a mixed solution of two or more of succinic anhydride modified adenine, succinic anhydride modified guanine, succinic anhydride modified cytosine, succinic anhydride modified thymine and succinic anhydride modified uracil is used. The functional molecule contains an ester group and a hydroxyl group, which can be used for DNA synthesis and cleavage. The ester group is a cleavage site used for cleaving DNA synthesized on the chip into a free solution-like oligo pool. The hydroxyl group contained in the functional molecule is a starting site for DNA synthesis. In some embodiments of the present invention, the hydroxyl group provided by the base monomer is used for DNA synthesis.

To sum up, direct current of the aniline substance (such as p-aminophenylacetic acid and p-phenylenediamine) is used to electrically promote grafting (facilitating industrial production) to form a stable covalent surface on a metal chip surface, and then a connecting arm and a functional molecule are used for modification to form a stable linker on the chip surface. The formation principle of the stable linker includes the following steps: (1) an aromatic amine molecule is diazotized to generate aromatic carbon radicals in the presence of hydrochloric acid and sodium nitrite by applying a constant direct current voltage, the aromatic carbon radicals and electrode materials are reacted to form covalent bonds and bonded; (2) by using the cross-linking reaction of a carboxyl and an amino group, the chip is immersed in a long-chain connecting arm molecule solution, thereby modifying a long-chain molecule, increasing the distance between the functional group and the chip, and reducing the steric hindrance in subsequent use; and (3) the functional molecule is then modified on the connecting arm, the functional molecule contains a hydroxyl group and an ester group, which facilitates the subsequent electrically promoted DNA synthesis and cleavage on the chip.

The present invention further provides the use of the chip surface linker described above in DNA synthesis or chip kit preparation. The chip surface linker of the present invention includes the following uses: DNA synthesis on a chip; disease biomarker chip detection; developing a chip kit of POCT (Point-of-Care-Testing); and high-throughput chip screening kit.

The chip surface linker in the present invention can also be used for DNA synthesis after hot water treatment, wherein the synthesized DNA is hybridized with oligonucleotides (such as a DNA primer) and then treated with base and hot TE; and the synthesized DNA can also be used for hybridization with oligonucleotides (such as a DNA primer). After the DNA synthesized by the chip surface linker of the present invention is subjected to repeated nucleic acid hybridization, elution, hybridization and recycle, the chip surface linker can still maintain good stability. The chip surface linker prepared in the embodiment of the present invention has good electrical conductivity and stability during energization, and can be used for electrically assisted synthesis of nucleic acid molecules such as DNA. If the linker does not have such properties, DNA synthesis will be affected. The chip surface linker of the present invention is resistant to an organic solvent required for DNA synthesis, otherwise DNA synthesis cannot be performed. When the chip linker is used for chip hybridization detection, the present invention can solve the problems that an existing linker is sensitive to water and unstable under heat, so the chip can be reused.

The term "chip" refers to a solid support formed of inorganic substances such as semiconductors or metals such as gold, silver and platinum, and the surface of the chip has a microarray formed by specific sites which are usually arranged in rows and lines, wherein each site can be used in some types of chemical or biochemical analysis, synthesis or method. These sites on the microarray are typically smaller than 100 microns. In the present invention, the chip is a platinum chip.

The term "linker" refers to a molecule, one end of which is or can be connected to a solid surface (e.g, a metal chip) and the other end of which has a reactive group, wherein the reactive group is or can be connected to a relevant chemical substance, such as small molecules, oligomers or polymers. The linker can be bonded to the solid surface, and/or a reactive group of the linker has been connected to the relevant chemical substance. The reactive group of the linker can be connected to a protecting group, wherein the protecting group can be removed chemically or electrochemically. The linker may contain a plurality of molecule groups, wherein the molecules are covalently connected.

The term "bonding molecule group" refers to a chemical molecule located at the end of the linker, one end of the group can be covalently connected to a solid surface (such as a metal chip), and the other end of the group has a reactive group, wherein the reactive group is or can be connected to a relevant chemical substance, such as small molecules, oligomers or polymers, and the reactive group can be connected to the connecting arm molecule or functional molecule in the present invention. The bonding molecule group has been bonded to the solid surface, and/or its reactive group has been connected to relevant chemical substances (e.g., a connecting arm molecule group or a functional molecule group). The reactive group of the bonding molecule group can be connected to a protecting group, wherein the protecting group can be removed chemically or electrochemically.

The term "connecting arm molecule group" refers to a chemical molecule located in the middle part of the linker, one end of the group can be connected to the bonding molecule group, and the other end of the group has a reactive group, wherein the reactive group is or can be connected to relevant chemical substances, such as small molecules, oligomers or polymers, and the reactive group can be connected to the functional molecule in the present invention. The connecting arm molecule group can be bonded to the bonding molecule group, and/or its reactive group has been connected to relevant chemical substances (e.g., a bonding molecule group or a functional molecule group). The reactive group of the connecting arm molecule group can be connected to a protecting group, wherein the protecting group can be removed chemically or electrochemically. The connecting arm molecule group can be formed in situ on the bonding molecule group. The connecting arm molecule group can be formed first, and then connected to the bonding molecule group that has been connected to the solid surface. The connecting arm molecule group can be externally synthesized on the relevant chemical substance first, and then connected to the bonding molecule group that has been connected to the solid surface. The relevant chemical substance can be connected to the connecting arm molecule group connected to the bonding molecule group, and then the entire structure can be connected to the reaction site on the solid surface. The purpose of the connecting arm molecule group is to extend the distance between the relevant molecules and the solid surface of the chip and reduce the steric hindrance in subsequent use. In the present invention, the connecting arm molecule group may be ethylenediamine group, hexamethylenediamine group, decanediamine group, 1,8-octanediamine group, ethylene glycol group, hexanediol group, decanediol group, 1,8-octanediol group and the like.

The term "functional molecule group" refers to a chemical molecule located in the terminal part of the linker, one end of the group can be connected to the connecting arm molecule group or the bonding molecule group, and the other end of the group has a reactive group, wherein the reactive group is or can be connected to relevant chemical substances, such as small molecules, oligomers or polymers, and in the present invention, the reactive group can be connected to deoxyribonucleotide molecules. The functional molecule group can be connected to the connecting arm molecule group and/or the reactive group to which relevant chemical substances are connected. The reactive group of the functional molecule group can be connected to a protecting group, wherein the protecting group can be removed chemically or electrochemically. In the present invention, the functional molecule may be succinic anhydride modified adenine, succinic anhydride modified guanine, succinic anhydride modified cytosine, succinic anhydride modified thymine or succinic anhydride modified uracil; hydroxyethyl methacrylate; succinic anhydride modified adenine, succinic anhydride modified guanine, succinic anhydride modified cytosine, succinic anhydride modified thymine or succinic anhydride modified uracil; and oxalic acid modified adenine, oxalic acid modified guanine, oxalic acid modified cytosine, oxalic acid modified thymine and oxalic acid modified uracil and the like.

The term "base monomer" refers to a molecule capable of polymerizing to form a macromolecule, such as a combined unit of the basic structure of an oligomer, a co-oligomer, a polymer or a copolymer. Examples of the monomer include A, C, T, G, adenylate, guanylate, cytidine, uridylate, amino acids, and other compounds.

The term "aryl" refers to an aromatic carbocyclic group having a monovalent value and about 4-20 carbon atoms. Examples of the aryl include but not limited to: phenyl, naphthyl and anthracenyl. One or more hydrogen atoms of a substituted aryl can be replaced with other groups. Although the aryl is monovalent by definition, the aryl as used herein includes groups having multiple valences, thereby meeting the requirement of substitution. The aryl can be part of a fused ring structure. For example, N-hydroxysuccinimide is bonded to phenyl (benzene) to form N-hydroxyphthalimide.

The term "aromatic amine molecule" refers to an amine with an aromatic substituent, that is, -NH2, -NH- or a nitrogen-containing group is connected to an aromatic ring, and the structure of the aromatic hydrocarbon generally contains one or more benzene rings. Aniline is the simplest example of such compounds. In the present invention, the aromatic amine molecule may be p-aminophenylacetic acid, p-aminophenylethanol or p-aminophenylenediamine.

The term "oligomer" refers to a molecule having intermediate relative molecular mass, the structure thereof basically contains a small number of units practically or conceptually derived from molecules having low relative molecular mass. If the properties of a molecule are indeed significantly different after removing one or several units, the molecule is considered to have an intermediate relative molecular mass. If part or the whole of the molecule has an intermediate relative molecular mass and basically contains a small number of units practically or conceptually derived from molecules having low relative molecular mass, the molecule can be described as oligomeric or oligomer used as an adjective. Oligomers are generally composed of monomers.

### Beneficial effects

Compared with the prior art, the present invention features that an aniline substance is effectively bonded to an electrode surface through carbon free radicals under the condition of constant direct current voltage, thereby forming a metal-carbon covalent bond to stably bond the molecule to the chip surface, and then the connecting arm molecule and the functional molecule are used for modification to form a stable linker on the chip surface. A chip surface linker with high adhesion is obtained in the present invention, which can be stably bonded to the chip surface. The chip surface linker of the present invention is stable in hot water and basic conditions, has good electrical conductivity, stability during energization and resistance to organic solvents required for nucleic acid synthesis, and is thus very advantageous for subsequent nucleic acid synthesis and other uses.

### Brief Description of the Drawings

FIG. 1 is a schematic diagram of the preparation of a chip surface linker and use thereof in DNA synthesis in Example 1 of the present invention, wherein (1) is a metal chip, (2) is the linker prepared by this method, and (3) is DNA synthesized on this linker;
FIG. 2 is a schematic diagram of the comparison of the stability of the chip surface linker prepared by the method in Example 1 of the present invention in hot water and that of a traditional linker;
FIG. 3 shows the stability of the chip surface linker prepared by the method in Example 1 of the present invention in nucleic acid synthesis;
FIG. 4 is a schematic diagram of hybridization experiment of the chip surface linker prepared by the method in Example 1 of the present invention after being used for DNA synthesis;
FIG. 5 is a schematic diagram of the cleavability of DNA synthesized by the chip surface linker prepared by the method in Example 1 of the present invention;
FIG. 6 is a schematic diagram of DNA synthesized by the chip surface linker prepared by the method in Example 1 of the present invention;
FIG. 7 is a gel electrophoresis diagram of DNA synthesized by the chip surface linker prepared by the method in Example 1 of the present invention, wherein the sample is DNA (120 nt) synthesized by the chip surface linker prepared in Example 1, and the control is DNA (120 nt) synthesized by the original linker chip disclosed in Example 1 of US 20060105355 A1, and ladder represents the standard (after electrophoresis, the positions of 50 nt, 75 nt, 150 nt, 200 nt and 300 nt nucleic acids can be displayed).

### Detailed Description of Embodiments

The technical solution in the examples of the present disclosure will be clearly and completely described below; obviously, the examples described are merely some, rather than all, of the examples of the present disclosure. On the basis of the embodiments of the present invention, all other embodiments obtained by those of ordinary skill in the art without involving any inventive effort fall within the scope of protection of the present invention.

In order to further understand the present application, the chip surface linker and a preparation method and use thereof provided by the present application will be described in detail below in combination with examples. However, it should be understood that these examples are implemented on the premise of the technical solution of the present invention, and the detailed embodiments and specific operation processes are given only to further illustrate the features and advantages of the present invention, but not to limit the claims of the present invention. The protection scope of the present invention is also not limited to the examples described below.

**Example 1:** Preparation of chip linker and use thereof in DNA synthesis Specific experimental steps:
1. chip cleaning: as shown in FIG. 1a, a platinum chip is sequentially rinsed with distilled water for 5 times, ethanol for 5 times and distilled water for 3 times, and then the chip is soaked in prinaha solution (the volume ratio of H₂SO₄ : H₂O₂ = 3 : 1), placed in an oven at 65°C for 30 min, and the chip is rinsed with distilled water for 5 times and blow-dried with argon;
2. chip modification: 18.15 mg cold p-aminophenylacetic acid is taken and mixed with 15 mM hydrochloric acid (1820 uL H₂O, 180 uL 0.5 M HCl) uniformly, 6.21 mg sodium nitrite is then added, and same is quickly shaken well;
3. this mixed liquid is quickly added onto the chip surface, 2.5 v of constant direct current voltage is applied to the chip, and the chip is left to stand for an electrically promoted reaction at room temperature for 20 min, and the chip modification reaction is as follows: 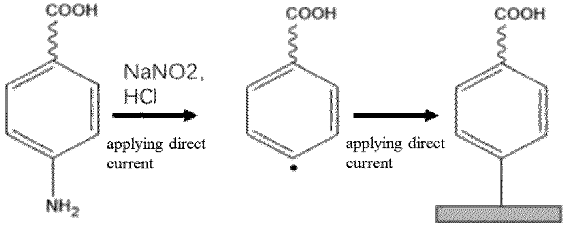
4. the chip is taken out and rinsed with distilled water for 5 times, ethanol for 5 times, 15 mM hydrochloric acid for 5 times and distilled water for 3 times, and then blow-dried with argon;
5. the chip is immersed in 8.7 mg of a solution of 1,8-octanediamine in methanol (1 mL methanol, 1.53 mg NHS and 7.64 mg EDC dissolved in 100 uL water, wherein NHS is N-hydroxysuccinimide and EDC is 1-ethyl-3-(3-dimethylaminopropyl)carbodiimide), and same is left to stand for reaction for 8 h, rinsed with distilled water for 5 times and blow-dried with nitrogen;
6. the chip is soaked in a succinic anhydride modified base monomer mixed solution (10.8 mg base monomer (adenine, thymine, guanine or cytosine), 1.53 mg NHS, 7.64 mg EDC dissolved in 100 uL water), and same is left to stand for reaction for 8 h;
7. the chip is sequentially rinsed with ethanol, acetone, ethanol and distilled water for 5 times and blow-dried with nitrogen to obtain this stable linker, as shown in FIG. 1b;
8. the linker is placed on a CustomArray chip synthesizer for DNA synthesis, as shown in FIG. 1c.

The schematic diagram of the linker prepared by the above steps is shown in FIG. 1d. The chip is sequentially connected to a bonding molecule, a connecting arm molecule and a functional molecule to obtain a chip linker, and the linker includes a bonding molecule group, a connecting arm molecule group and a functional molecule group. The prepared linker is then used for DNA synthesis (using the CustomArray chip synthesizer), the schematic diagram of the obtained effect is shown in FIG. 1c, and perfect DNA can be synthesized.

### Example 2: Comparison of stability of new and old linkers in hot water

After the linker is modified on the chip, it is placed in distilled water at 80°C for 2 days, followed by DNA synthesis.

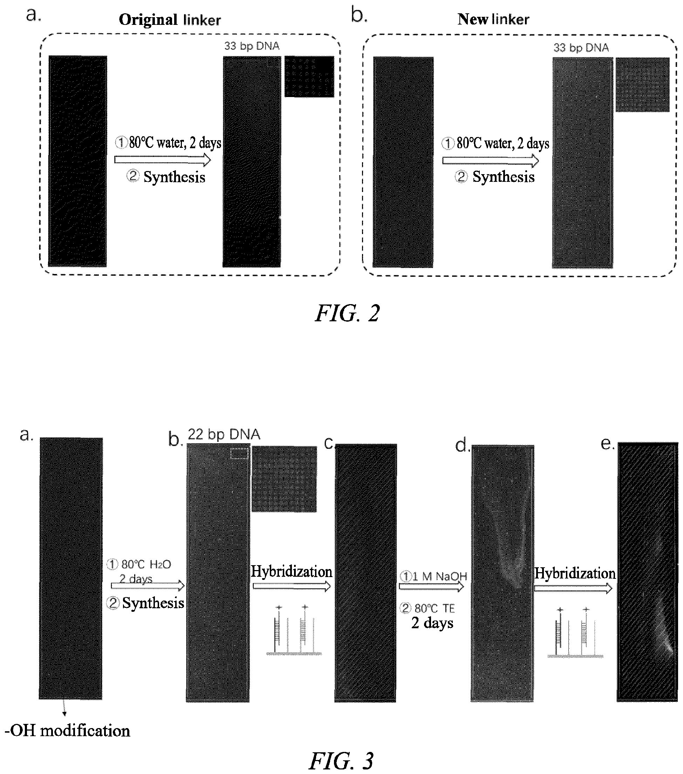

For an original linker, a chip is deposited with polyhydroxy micromolecules (see Example 1 in US 20060105355 A1), rinsed with distilled water and dried, and then placed in distilled water at 80°C for 2 days and blow-dried with argon, then 33 nt DNA is synthesized on the chip by using a CustomArray chip synthesizer, and the chip is scanned on a chip scanner (CustomArray, GenePix4000B). As shown in FIG. 2a, DNA synthesis is impossible in most areas.

The new linker is prepared according to the method in Example 1 of the present invention. For the new linker, a chip is placed in distilled water at 80°C for 2 days and blow-dried with argon, then 33 nt DNA is synthesized on the chip by using a CustomArray chip synthesizer, and the chip is scanned on a chip scanner (CustomArray, GenePix4000B). As shown in FIG. 2b, the DNA on the chip surface can still exist stably, indicating that the new linker has sufficient stability in hot water, which is of great value to the subsequent high-temperature hybridization of the chip surface or other detection applications.

### Example 3: Stability of new linker in nucleic acid synthesis application

After hot water treatment and DNA synthesis, the new linker is subjected to hot TE and basic solution treatment and then used for hybridization.

The new linker is prepared according to the method in Example 1 of the present invention. For the new linker, a chip is placed in distilled water at 80°C for 2 days and blow-dried with argon, then 33 nt DNA is synthesized on the chip by using a CustomArray chip synthesizer, and the chip is scanned on a chip scanner. The results are shown in FIGs. 3a and 3b. Two DNA primers with fluorescence (100 pM each) are taken and hybridized with the DNA synthesized on the chip (at room temperature, hybridization reaction for 2 h), and the chip is rinsed with PBS buffer and scanned on a chip scanner. As shown in FIG. 3c, different fluorescent spots are displayed, which indicates that the two DNA primers can be hybridized with the DNA synthesized on the chip. The chip is then rinsed with 1 M NaOH solution to remove the hybridized primer, placed in TE buffer (a mixed solution of 5 mL of 1 M Tris-HCl Buffer with pH 8.0 and 1 mL of 0.5 M EDTA with pH 8.0,) at 80°C for 2 days, blow-dried with argon, and then scanned on a chip scanner. The result is shown in FIG. 3d. Two DNA primers with fluorescence (100 pM each) are taken and hybridized with the DNA synthesized on the chip (at room temperature, hybridization reaction for 2 h), and the chip is rinsed with PBS buffer and scanned on a chip scanner. As shown in FIG. 3e, different fluorescent spots are displayed, which indicates that the two DNA primers can be hybridized with the DNA synthesized on the chip. It indicates that the new linker has sufficient stability in hot water and is resistant to alkali and hot TE, which is of great value for the subsequent high-temperature hybridization of the chip surface or other detection applications.

### Example 4: Stability of new linker in repeated hybridization-elution-hybridization applications

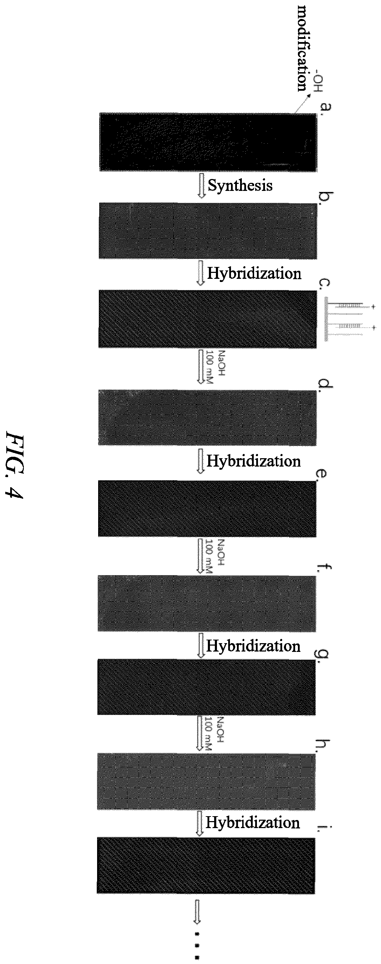

The new linker is prepared according to the method in Example 1 of the present invention. As shown in FIG. 4, after a chip is blow-dried with argon, 33 nt DNA is synthesized on the chip by using a CustomArray chip synthesizer, and the chip is scanned on a chip scanner. The result is shown in FIG. 4b. Two DNA primers with fluorescence (100 pM each) are taken and hybridized with the DNA synthesized on the chip (at room temperature, hybridization reaction for 2 h), and the chip is rinsed with PBS buffer and scanned on a chip scanner. As shown in FIG. 4c, different fluorescent spots are displayed. The chip is then rinsed with 100 mM NaOH solution to remove the hybridized primer, blow-dried with argon and scanned on a chip scanner. The result is shown in FIG. 4d. Two DNA primers with fluorescence(100 pM each) are taken and hybridized with the DNA synthesized on the chip (at room temperature, hybridization reaction for 2 h), and the chip is rinsed with PBS buffer and scanned on a chip scanner. As shown in FIG. 4e on the right, after multiple cycles, the chip can still be used for hybridization and has obvious fluorescence signals, which indicates that this linker is stable enough to withstand repeated hybridization and alkali-solution elution.

### Example 5: Cleavability of synthesized DNA from the chip

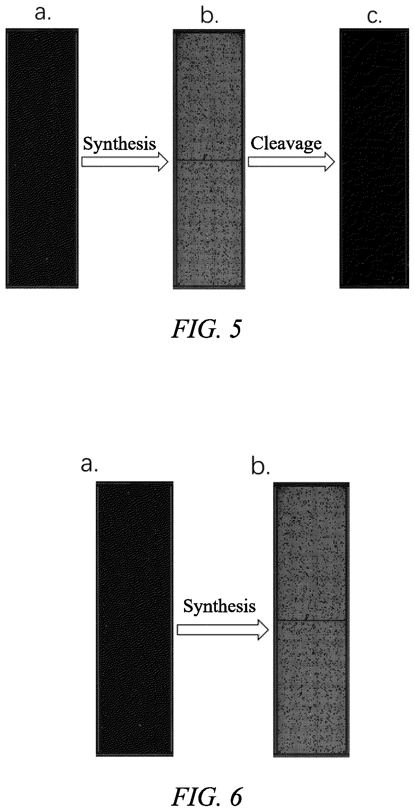

The linker (FIG. 5a) is prepared by the method in Example 1 of the present invention, and then 120 nt DNA is synthesized on the chip by a CustomArray chip synthesizer (FIG. 5b). The formed linker contains a cleavage group (an ester group), which can cleave oligonucleotide (oligo) from the chip (16 h) under basic (ammonia) and heating condition (65°C) to form free oligo. The chip is rinsed with water and then scanned by using a chip scanner (FIG. 5c), which indicates that the whole DNA is basically cleaved.

### Example 6: DNA quality characterization of new linker for DNA synthesis

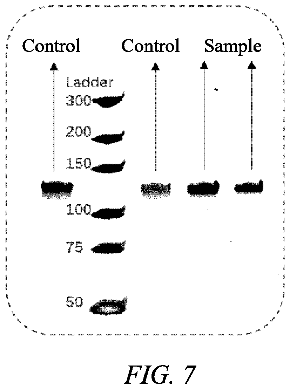

The linker is prepared by the method in Example 1 of the present invention, and then 120 nt DNA is synthesized on the chip by using a CustomArray chip synthesizer (as shown in FIG. 6). The formed linker contains a cleavage group (an ester group), which can cleave oligonucleotide (oligo) from the chip (16 h) under basic (ammonia) and heating condition (at 65°C) to form a free oligonucleotide pool (oligo pool). The concentrations of the two chips determined by Thermo Scientific^{™} NanoDrop^{™} One Microvolume UV-Vis Spectrophotometers are 21.4 ng/uL and 19.4 ng/uL, which meets the requirements. Next, PCR amplification is carried out, and the resulting product is subjected to gel electrophoresis diagram analysis. As shown in FIG. 7, the size of the resulting product is similar to that of the standard product, and they are located at the same position, which indicates that the product synthesized by such linker is correct.

### References:

1. Sharma R. Small-molecule Surfactant Adsorption, Polymer Surfactant Adsorption, and Surface Solubilization: An overview [M]. 1995.
2. Kokkin D L, Zhang R, Steimle T C, et al. Au-S Bonding Revealed From the Characterization of Diatomic Gold Sulfide, AuS[J]. The Journal of Physical Chemistry A, 2015, 119(48) : 11659 - 11667.
3. Toma M, Tawa K. Polydopamine Thin Films as Protein Linker Layer for Sensitive Detection of Interleukin-6 by Surface Plasmon Enhanced Fluorescence Spectroscopy [J]. ACS Applied Materials & Interfaces, 2016, 8(34) : 22032 - 22038.
4. Saaem I, Ma K S, Marchi A N, et al. In Situ Synthesis of DNA Microarray on Functional Cyclic Olefin Copolymer Substrate[J]. ACS Applied Materials & Interfaces, 2010, 2(2) : 491 - 497

## Claims

1. A chip surface linker, **characterized in that** the linker is prepared by the following steps:
step 1: applying a direct current voltage to cause an aromatic amine bonding molecule to react with a chip surface in the presence of an acid and a nitrite to form a bonding molecule group connected to the chip surface; and
step 2: using a functional molecule for reaction and modification to obtain a linker containing a functional molecule group, wherein the functional molecule group contains a hydroxyl group and an ester group.

2. The chip surface linker according to claim 1, **characterized in that** the bonding molecule is an aniline substance.

3. The chip surface linker according to claim 1 or 2, **characterized in that** the bonding molecule is selected from p-aminophenylacetic acid, p-aminophenylethanol or p-aminophenylenediamine, preferably p-aminophenylacetic acid.

4. The chip surface linker according to any one of claims 1 to 3, **characterized in that** the direct current voltage is a constant direct current voltage, and the direct current voltage applied is selected from 0.5 to 5.0 V, preferably 0.5 to 3.0 V.

5. The chip surface linker according to any one of claims 1 to 4, **characterized in that** the direct current voltage is applied for 10 to 50 min, preferably 10 to 30 min.

6. The chip surface linker according to any one of claims 1 to 5, **characterized in that** the nitrite is selected from sodium nitrite, potassium nitrite or calcium nitrite, preferably sodium nitrite.

7. The chip surface linker according to any one of claims 1 to 6, **characterized in that** the acid is selected from hydrochloric acid, nitric acid or sulfuric acid, preferably hydrochloric acid.

8. The chip surface linker according to any one of claims 1 to 7, **characterized in that** the functional molecule is a hydroxyl substance containing a long carbon chain and an ester group.

9. The chip surface linker according to claim 8, **characterized in that** the functional molecule is selected from a succinic anhydride modified base monomer, hydroxyethyl methacrylate, a succinic acid modified base monomer or an oxalic acid modified base monomer, preferably a succinic anhydride modified base monomer.

10. The chip surface linker according to claim 9, **characterized in that** the base monomer in the functional molecule is selected from one or more of adenine, guanine, cytosine, thymine and uracil.

11. The chip surface linker according to any one of claims 1 to 10, **characterized in that** a connecting arm molecule group is further included between the bonding molecule group and the functional molecule group.

12. The chip surface linker according to claim 11, **characterized in that** the connecting arm molecule group is reacted with and connected to the bonding molecule group by a connecting arm molecule, and the functional molecule group is reacted with and connected to the connecting arm molecule group by a functional molecule.

13. The chip surface linker according to claim 12, **characterized in that** the connecting arm molecule is a diamine or diol substance.

14. The chip surface linker according to claim 12 or 13, **characterized in that** the connecting arm molecule is selected from ethylenediamine, hexamethylenediamine, decanediamine, 1,8-octanediamine, ethylene glycol, hexanediol, decanediol or 1,8-octanediol, preferably 1,8-octanediamine.

15. The chip surface linker according to any one of claims 1 to 14, **characterized in that** the chip is a metal chip, preferably a gold chip, a platinum chip or an aluminum chip.

16. The chip surface linker according to claim 15, **characterized in that** the chip is a metal platinum chip.

17. A preparation method of a chip surface linker, **characterized by** comprising the following steps:
step 1: mixing an aromatic amine bonding molecule with an acid and a nitrite to obtain a mixed solution;
step 2: bringing the mixed solution in step 1 into contact with a chip surface, and applying a direct current voltage for reaction to form a bonding molecule group connected to the chip surface; and
step 3: reacting the reacted chip surface with a functional molecule to obtain a linker containing a functional molecule group, wherein the functional molecule group contains a hydroxyl group and an ester group.

18. The preparation method according to claim 17, **characterized in that** before step 3, the preparation method comprises bringing the reacted chip surface in step 2 into contact with a connecting arm molecule for reaction, so as to connect a connecting arm molecule group.

19. The preparation method according to claim 17 or 18, **characterized in that** the bonding molecule is an aniline substance.

20. The preparation method according to any one of claims 17 to 19, **characterized in that** the bonding molecule is selected from p-aminophenylacetic acid, p-aminophenylethanol or p-aminophenylenediamine, preferably p-aminophenylacetic acid.

21. The preparation method according to any one of claims 17 to 20, **characterized in that** the direct current voltage is a constant direct current voltage, and the direct current voltage applied is selected from 0.5 to 5.0 V, preferably 0.5 to 3.0 V.

22. The preparation method according to any one of claims 17 to 21, **characterized in that** the direct current voltage is applied for 10 to 50 min, preferably 10 to 30 min.

23. The preparation method according to any one of claims 17 to 22, **characterized in that** the nitrite is selected from sodium nitrite, potassium nitrite or calcium nitrite, preferably sodium nitrite.

24. The preparation method according to any one of claims 17 to 23, **characterized in that** the acid is selected from hydrochloric acid, nitric acid or sulfuric acid, preferably hydrochloric acid.

25. The preparation method according to any one of claims 17 to 24, **characterized in that** the functional molecule is selected from a succinic anhydride modified base monomer, hydroxyethyl methacrylate, a succinic acid modified base monomer or an oxalic acid modified base monomer, preferably a succinic anhydride modified base monomer.

26. The preparation method according to claim 25, **characterized in that** the base monomer in the functional molecule is selected from one or more of adenine, guanine, cytosine, thymine and uracil.

27. The preparation method according to any one of claims 17 to 26, **characterized in that** the connecting arm molecule is a diamine or diol substance.

28. The preparation method according to claim 27, **characterized in that** the connecting arm molecule is selected from ethylenediamine, hexamethylenediamine, decanediamine, 1,8-octanediamine, ethylene glycol, hexanediol, decanediol or 1,8-octanediol, preferably 1,8-octanediamine.

29. The preparation method according to any one of claims 17 to 28, **characterized in that** when the direct current voltage is applied for reaction in step 2, the temperature is 4°C to 40°C, preferably 20°C to 37°C; and the reaction time is 10 to 50 min, preferably 10 to 30 min.

30. The preparation method according to any one of claims 17 to 29, **characterized in that** the chip is a metal chip, preferably a gold chip, a platinum chip and an aluminum chip.

31. Use of the chip surface linker according to any one of claims 1 to 16 in nucleic acid synthesis or chip kit preparation.
